(19)

Europäisches
Patentamt
European
Patent Office
Office européen
des brevets

(11) **EP 3 171 173 A1**

(12) **EUROPEAN PATENT APPLICATION**

(43) Date of publication:
**24.05.2017 Bulletin 2017/21**

(51) Int Cl.:
*G01N 33/68* (2006.01)  *G01N 33/92* (2006.01)

(21) Application number: **15195509.3**

(22) Date of filing: **19.11.2015**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **Medizinische Universität Innsbruck 6020 Innsbruck (AT)**

(72) Inventors:
• **Dumfarth, Julia 6020 Innsbruck (AT)**

• **Grimm, Michael 6020 Innsbruck (AT)**
• **Bernhard, David 6300 Wörgl (AT)**
• **Doppler, Christian 6020 Innsbruck (AT)**

(74) Representative: **Schwarz & Partner Patentanwälte OG**
**Patentanwälte**
**Wipplingerstraße 30**
**1010 Wien (AT)**

(54) **METHOD OF DIAGNOSIS OF A THORACIC AORTIC ANEURYSM**

(57)      Method for diagnosing a thoracic aortic aneurysm comprising the steps of
a) quantifying a metabolite level based on at least one metabolite in a sample from a subject,
b) comparing the metabolite level in the sample with a reference value, wherein the reference value is obtained by quantifying the metabolite level determined in step a) in a sample from at least one healthy control subject, and
c) determining if there is a difference of the metabolite level to the reference value, wherein a difference between the metabolite level and the reference value indicates a higher risk of prevalence of a thoracic aortic aneurysms in the subject.

Fig. 1

**Description**

FIELD OF THE INVENTION

The present invention relates to method of diagnosis of a thoracic aortic aneurysm and a kit for diagnosis of a thoracic aortic aneurysm.

DESCRIPTION OF RELATED ART

In general, aneurysms represent a Damocles sword in the classical sense for patients diagnosed with this disease and a hidden version of the sword for the general population. In the past decade the efforts to understand the pathogenesis of aneurysms has increased significantly. This knowledge has led to important developments in experimental treatment, early diagnosis, and risk assessment combined with improved and more personalized handling of patients. Despite the progress made our understanding of the pathogenesis of aneurysms in general and the thoracic aortic aneurysm (TAA) in particular is still poor. 51% of all aneurysms affecting the thoracic aorta are related to the ascending part of the aorta, i.e. ascending thoracic aortic aneurysm (ATAA).

Importantly, genetic disorders with a high risk for the occurrence of ATAA and their genetic basis, like the Marfan syndrome (fibrillin-1 defects), Loeys-Dietz syndrome (TGF-$\beta$ 1/2 defects), the vascular form of the Ehlers-Danlos syndrome (type III collagen defects) are well described. Also the Turner syndrome is associated with an increased risk for ATAAs as well as cardiac anomalies (e.g. a bicuspid aortic valve). 0.5 - 1.4 % of the general population have bicuspid aortic valves (BAV), which are associated with an increased risk for ATAAs. Despite being clearly a congenital disorder, the genetic basis for BAV development is less clear determined compared to the above. It is already known that BAVs are clearly associated with Turner syndrome (X0) and male gender (XY) suggesting an X-linked genetics. But, recent results also suggest autosomal linkage. Gene mutations that were found to be associated with BAVs affect NOTCH1, GATA5, TGFBR1 and TGFBR2. In recent years also family-linked forms of the ATAA have been described. Importantly, the majority of ATAAs is of unknown origin.

Besides BAV-associated thoracic aortic aneurysm (BAV), TAA can also be associated with an aortic valve showing the normal number of tree cusps, i.e. a tricuspid aortic valve-associated thoracic aortic aneurysm (TAV).

Another category of an thoracic aortic disease is a dissection of the aorta. In contrast to a rupture of the aorta, the adventitia (or *tunica externa*) of the aorta remains intact, but it comes to blood penetration through the intima (or *tunica intima*) and blood enters into the media (or *tunica media*). Aneurysms are often preceding or associated with a thoracic aortic dissection. A typical clinical picture is an thoracic aortic dissection (Diss).

Studies on the pathogenesis of non-syndromic ATAAs are based on two major histological findings in TAA tissue, i.e. the loss of smooth muscle cells (medial degeneration) and the alteration of elastic fibre structures. Note that despite recent consensus in the literature on these phenotypes, older work - which addressed these issues in detail - are less uniform.

The central and major risk factor for ATAA formation (apart from genetic factors) is hypertension. Smoking, atherosclerosis, and inflammation have an influence on abdominal aortic aneurysms (AAA) formation; however these are less relevant in ATAAs. Most factors analysed so far and found to have some impact on ATAA formation originate from previous studies on AAA patient samples or from the broader field of cardiovascular sciences. They mostly include the MMP2/9 - TIMP system, smooth muscle stress and cell death, aging processes (telomere length), and alterations in genes and protein expression and function of e.g. SOD, AKT, SMAD, ostepontin, angiotensin and the factors mentioned above (genetic disorders).

At present TAA is diagnosed purely by chance and only with imaging techniques. Reliable clinical markers for the early detection, disease stages and progression of ATAAs are not routinely applied. The "omic" technologies in general offer great opportunities for the discovery of pathophysiologically relevant factors which can also be used as disease markers and markers for an improved stratification of diseases. These markers - given their involvement in relevant pathophysiological processes can be demonstrated in further studies - may also serve as therapeutic targets in the future. Generally, despite great progress in ATAA "omics" in recent years, this area is still at its beginning.

The "omics" era in aneurysm research started with a genomic analysis of intracranial aneurysm by Peters et al. (Peters DG, Kassam AB, Yonas H, O'Hare EH, Ferrell RE, Brufsky AM. Comprehensive transcript analysis in small quantities of mRNA by SAGE-lite. Nucleic acids research. 1999; 27: e39.) followed by the first transcriptome analyses in AAA tissues by Tung et al. who reveal 44 genes that were differentially expressed between controls and AAA and suggested a pro-inflammatory, matrix degrading, pro-atherosclerotic, and smooth muscle cell depleting profile (Tung WS, Lee JK, Thompson RW. Simultaneous analysis of 1176 gene products in normal human aorta and abdominal aortic aneurysms using a membrane-based complementary DNA expression array. Journal of vascular surgery. 2001; 34: 143-50. 10.1067/mva.2001.113310).

In WO 2010/127381, in human studies it was shown that components of the kallikrein-kinin system are associated with AAA. Besides methods for preventing or treating an aneurysm by administering an antagonist of the kallikrein-kinin system, a method for detecting the presence of an aneurysm, determining the prognosis of an aneurysm, or monitoring the progression of an aneurysm is disclosed, wherein altered levels of gene expression or protein activity of a component of the kallikrein-kinin system correlates with the presence or risk of the condition.

Regarding AAA, also metabolomic studies have been performed. The first analyses of the metabolome of aortic aneurysms (AAA) was published by the team around Ciborowski and Barbas (Ciborowski M, Martin-Ventura JL, Meilhac O, Michel JB, Ruperez FJ, Tunon J, Egido J, Barbas C. Metabolites secreted by human atherothrombotic aneurysms revealed through a metabolomic approach. Journal of proteome research. 2011; 10: 1374-82. 10.1021/pr101138m). Ciborowski et al. could reveal increased levels of the lipid class of lyso phosphatidyl cholines (PCs) in the secretome of in vitro incubated AAA samples compared to controls. Further the authors describe alterations in amino acid metabolism and suggest that the analysis of enzymes relevant to catabolism and anabolism may reveal novel insights (Ciborowski M, Barbas C. Metabolites secreted by human atherothrombotic aneurysm. Methods in molecular biology. 2013; 1000: 103-13. 10.1007/978-1-62703-405-0_9). In another study by the same team, Ciborowski et al demonstrate that plasma metabolomic analyses has the potential to predict AAA disease stages (Ciborowski M, Teul J, Martin-Ventura JL, Egido J, Barbas C. Metabolomics with LC-QTOF-MS permits the prediction of disease stage in aortic abdominal aneurysm based on plasma metabolic fingerprint. PloS one. 2012; 7: e31982. 10.1371/journal.pone.0031982).

Based on an analysis of urine of mice with AAA, WO 2009/091581 suggests a method for diagnosing and staging aneurysms via biomarkers comprising an elastin degradation product and a collagen degradation product, such as desmosine and pyridinoline.

Alternative methods for diagnosing disease of aorta based on a biomarker are disclosed in WO 2007/103568. It was shown that specific protein or polypeptide markers can distinguish subjects with an aortic dissection from healthy control subjects. Based on the description of the examples it can not be determined if the aortic dissection is associated to an abdominal or thoracic aneurysm.

While most studies identifying suitable markers based on AAA, less approaches are related to TAA. The first study on aneurysm proteome by Matt et al. was published in 2007 and analysed ATAA samples (Matt P, von Orelli A, Bernet F, Grussenmeyer T, Lefkovits I, Zerkowski HR. Proteomics of ascending aortic aneurysm with bicuspid or tricuspid aortic valve. Asian cardiovascular & thoracic annals. 2007; 15: 185-90). In this study the authors compared the proteome of aortic valve or aorta surgery patients with tricuspid aortic valves (TAV) or bicuspid aortic valves (BAV). Genetic biomarkers for identification of ATAA in a whole blood sample are investigated based on a proteomic analysis of expressed proteins in tissue samples (Black KM, Masuzawa A, Habgerg RC, Khabbaz KR, Trovato ME, Rettagliati M, Bhasin MK, Dillon ST, Libermann TA, Tournpoulis IK, Levitsky S, McCully JD. Preliminary biomarkers for identification of human ascending thoracic aortic aneurysm. Journal of The Amercian Heart Association. 2013; 2: 10.1161/JAJA.113.000138).

In WO 2014/004889 A2 a method for diagnosing and a method for monitoring TAA based on assaying levels of microRNAs, MMPs, TIMPs, or a combination thereof, in a blood or plasma sample is described.

In summary, present approaches for TAA relate to proteomics and metabolomic data on ATAA are not available. The current standard for diagnosis of thoracic aorta aneurysm is still relying on medical imaging only. Thus, the respective diagnosis must rely on appropriate technical infrastructure and eventually also mobility of analysed subject to access the infrastructure. Moreover, the imaging techniques are expensive and not appropriate for early diagnosing and broad scale screens.

## SUMMARY OF THE INVENTION

An object of the invention is to provide a method for assessing the risk of prevalence of a thoracic aortic aneurysms in the subject, which overcomes the previously mentioned problems with imaging techniques.

This problem is solved by a method for diagnosis of a thoracic aortic aneurysm comprising the steps of

a) quantifying a metabolite level based on at least one metabolite in a sample from a subject,
b) comparing the metabolite level in the sample with a reference value, wherein the reference value is obtained by quantifying the metabolite level determined in step a) in a sample from at least one healthy control subject, and
c) determining if there is a difference of the metabolite level to the reference value, wherein a difference between the metabolite level and the reference value indicates a higher risk of prevalence of a thoracic aortic aneurysms in the subject.

In another aspect the invention provides a method for typing of a thoracic aortic aneurysm comprising the steps of

a) quantifying a metabolite level based on at least one metabolite in a sample from a subject,
b) comparing the metabolite level in the sample with reference values compiled in a classifier system, wherein the classifier system is based on at least one reference value, wherein each reference value is obtained by quantifying the metabolite level determined in step a) in a sample from at least one control subject showing the thoracic aortic aneurysm of a single diagnostic type, and

c) determining the diagnostic type of the thoracic aortic aneurysm based on the classifier system

wherein the diagnostic type is selected out of bicuspid aortic valve-associated aneurysm (BAV), tricuspid aortic valve-associated aneurysm (TAV), or a dissection of the thoracic aorta (Diss).

A great advantage of the methods according to the invention in comparison to imaging techniques for diagnosis relates to the fact that the method according to the invention is based on a sample taken and isolated from the investigated subject. Thus, there is no need for respective imaging infrastructure or even invasive investigations. For example, blood samples, are routinely available from patients. A method based on blood samples has a high compliance and is applicable for a large scale of test subject (e.g. broad scale screens). Moreover, it is believed, that the method according to the invention is particularly appropriate for early diagnosis, e.g. in a state of progression where other methods might not have been considered due to lack of indicative symptoms or in a state where the aneurysm might not (yet) have detectable dimensions.

The term "thoracic aortic aneurysm" (TAA) according to the invention is defined as a disease of the thoracic section of the aorta including an enlargement or dilation of the aorta. This definition covers an "*aneurysm verum*" involving all three layers (*intima, media, adventitia*) of the aorta wall as well as a "*aneurysma dissecans*", i.e. an aortic dissection resulting in enlarged aorta due to blood penetration between the tunica intima and media of the aortic wall. According to the invention a thoracic aortic aneurysm comprises diseases falling into class I71 of the 10th revision of the International Statistical Classification of Diseases and Related Health Problems (ICD-10). Preferably, a thoracic aortic aneurysm comprises disease falling into the group comprising ICD-10 I71.0 (Dissection of aorta), I71.1 (Thoracic aortic aneurysm, ruptured), and I71.2 (Thoracic aortic aneurysm, without mention of rupture). According to the invention an thoracic aortic aneurysm should not include abdominal aortic aneurysms (AAA). Thoracic aortic aneurysm according to the invention may effect the ascending part of the thoracic aorta as well as the descending part of the aorta. Preferably, the method according to the invention is used for diagnosing a thoracic aortic aneurysm of the ascending part of the aorta.

The method for typing a TAA enables to differentiate between a thoracic aortic dissection and *aneurysma verum* of the thoracic aortic wall. Moreover, the method also allows to distinguish between thoracic aortic aneurysm associated with a tricuspid aortic valve (TAV) and TAA associated with bicuspid aortic valve (BAV). Bicuspid aortic valves, the most common cardiac valvular anomaly, is often associated with thoracic aortic aneurysm which might be rationalized by altered hemodynamics in the aorta. Different pathogenesis between BAV and TAV may have an implication on the selection of therapy. Thus, the method for typing a TAA into one of the respective subtypes of thoracic aortic aneurysm is not only of interest in respect of studying pathogenesis of aortic aneurysms but also implies on the subsequent medical treatment of the respective subject.

In the examples, an analysis of pooled metabolites from patient samples with different diagnostic background showed that significant differences in the relative level of metabolites are found for the individual diseases. These findings suggest that there is a valid basis for a method of diagnosing of TAA and typing of the diagnoses TAV, BAV, and Diss based on metabolites levels from a sample. Furthermore, for a biomarker based on selected sphingomyelin concentrations differentiation of BAV and Diss versus the control group could be achieved.

Regarding the recently proposed biomarkers for diagnosing TAA, the present invention has the advantage that according to the present invention the biomarker is a metabolite. Metabolites are normally defined as organic molecules occurring as products, by-products or intermediates of the metabolism. One definition defining a metabolite is usually referring to a molecules with less than 1 kDa in weight. However, according to the present invention metabolites should be understood as organic compounds excluding nucleic acids and proteins. The chemical variability of the metabolites allows alternative detection and quantification techniques. For some metabolites techniques for routine quantification starting from a patient sample are already established and might become routine approach for medical check-up. Thus, the method for diagnosing and typing of TAA based on metabolites provides a different approach in comparison to genomics or proteomics, wherein specific detection methods might be necessary.

Metabolites for which a metabolite level is quantified according to the present invention are metabolites, such as, but not limited to, amino acids, lipids such as glycerophospholipids, sphingolipids (as sphingomyelins or ceramides), acylcarnitines, monosaccharides, prostaglandins, hydroxyeicosatetraenoic acids, hydroxyoctadecadienoic acids, steroids, or bile acids.

Suitable metabolites for a method according to the invention can be determined as shown in example 1. Generally, a person skilled in the art can identify a suitable metabolite from various metabolites by quantifying them from samples by a patients group with thoracic aorta anomalies and compare it to values obtained for samples of a healthy control group.

The reference value used in a method for diagnosing a TAA according to the invention is derived from a healthy control subject or preferably a plurality of healthy control subjects representing a control group. The reference value for a metabolite level allows to evaluate the metabolite level of the analysed subject. The difference is regarded as relevant for indicating a higher risk of prevalence of a thoracic aortic aneurysms in the subject either in case the metabolite level is higher than the reference value or in case the metabolite level is lower than the reference value. The reference value preferably is chosen such that metabolite levels higher than the reference value indicate presence or a higher risk of prevalence of a TAA in the subject.

Preferably, the reference value is predetermined, e.g. during identification of suitable metabolite levels. Thus, no control subjects are needed to actually perform the method according to the invention. The difference of the metabolite level to the reference value can be determined by subtracting the two values. The reference value as well may be provided in a kit for performing the method of diagnosis of a thoracic aortic aneurysm according to the invention.

For a method for typing of a TAA according to the invention a classifier system is derived from a reference value. A method which allows to type a single diagnostic type may rely on a classifier system based on a single reference value. However, multiple reference values are needed to compile a classifier system for differentiating between more than one diagnostic type. For example multiple reference values for a metabolite level might build up quantitative ranges, wherein each range is associated to a diagnostic type. Preferably, a classifier system also includes an endpoint representing healthy subjects, i.e. a method for diagnosing and a method for typing TAA are combined. Exemplarily, a method relying on a classifier system may be based on a single metabolite level, and three reference values allowed for diagnosing of TAA and typing of the diagnostic classes TAV, BAV, and Diss. Alternatively, a decision tree classification system may be proposed based on more than one metabolite level. Alternatively, different statistical methods, like principle component analysis or partial least square analysis can be envisaged for enabling a suitable classifier system for diagnosing or typing when multiple metabolite levels (i.e. a metabolite profile) are used for distinguishing between diagnostic subtypes.

In a preferred embodiment according to the invention, the at least one metabolite is selected out of the group comprising representatives out of the classes amino acids, glycerophospholipids, sphingomyelins or ceramides.

The term "metabolite level" refers to a quantitative value for a metabolite. The metabolite level may be an amount or a concentration. According to the invention a metabolite level is quantified based on at least one metabolite. Thus, one or more compounds might contribute to a single value.

On one side the combination of different compounds into a single metabolite level can be the result of a quantification or detection method that does not distinguish between these compounds. Two similar but still chemically different compounds might be detected and/or quantified simultaneously resulting in a single metabolite level. Exemplary, different configurational isomers might not be distinguishable by mass spectrometry.

On the other side the inventors found that deliberately summing up more than one individually detected metabolite values into a common metabolite level resulted in markers with significant differences between control subjects and three patient groups of different diagnoses aortic disease.

Especially, the pooling of patients and analysis of metabolites in the class amino acids, phosphoglycerolipids, sphingomyelins, or ceramides showed that the metabolites for the diagnosis differ in a typical pattern. For sphingomyelins it could be shown that summing up specific metabolite levels allows to different patients with specific disease. Thus, the metabolite level based on summing up sphingomyelin levels provided a suitable method for diagnosing specific TAA disease.

Thus, in a further preferred embodiment according to the invention, the metabolite level of at least one metabolite results from summing up the individual levels of two or more metabolites.

Accordingly, it is preferred that the individual levels of two or more metabolites are selected out of a single specific class of metabolites, wherein the specific class is selected out the group comprising amino acids, glycerophospholipids, sphingomyelins or ceramides. Especially, when combining quantitative values from two or more metabolites to a metabolite level, it may be useful to normalize the individual metabolites levels before building a combined metabolite level. This can be achieved, for example, by dividing each measured metabolite level by a normalization value which should be determined based on a control group of healthy subjects. With a normalization individual metabolites with different concentration ranges in the sample will make the same quantitative contributions to a combined metabolite level.

In the context of metabolite levels from summing up individual levels, it should be mentioned that the technique used to quantify the metabolite level might be chosen to allow a common detection or quantification for a compound class simultaneously without differentiation of multiple individual compounds.

Generally, various different analytical approaches for quantifying the metabolite in step a) of the methods according to the invention can be applied. The approach may be selected from targeted metabolomics or untargeted metabolomics (profiling). Exemplarily, suitable quantification methods may be selected out of the group comprising flow injection analysis (FIA), chromatographic separation techniques: liquid chromatography (LC), gas chromatography (GC), capillary electrophoresis (CE), thinlayer chromatography (TLC) in combination with appropriate detection techniques, i.e. mass analyzer, UV spectroscopy detection, colorimetric detection, fluorescence detection and nuclear magnetic resonance (NMR). Preferably, a mass analyzer or spectrometry tool is selected out of the group comprising quadrupole mass analyzer, ion trap mass analyzer, time of flight (TOF) mass analyzer, orbitrap mass analyzer, magnetic sector mass analyzer, electrostatic/magnetic sector mass analyzer, fourier transform ion cyclotron resonance (FTICR) mass analyzer, single quadrupole (Q), and combinations like triple quadrupole (QqQ), QqTOF, TOF-TOF, Q-orbitrap.

Alternatively, also chemical detection approaches can be applied for an analytical step. For example, the reaction of amino acids with ninhydrin allows to detect and quantify amino acids and provides a potential analytical approach for quantifying the metabolite level for multiple amino acids in a single test. Thus, it is considered applicable for quantification step a) in methods according to the invention. Alternatively the method of detection may be biochemically .g. antibody-based techniques immuno-assays.

Preferably, a mass spectrometric approach is used in the methods according to the information. For example, the methods rely on a metabolite quantification approach as disclosed in US 2007/0004044. The method allows an efficient parallel detection and quantification of multiple metabolites.

The parallel or subsequent detection of more than one metabolite level results in a metabolite profile. Methods including the step of quantifying a metabolite profile rely on the basic principle of the invention according to which one ore more metabolite marker allow for diagnosing and/or typing of TAA.

The methods as previously described may be adapted in that in step a) two or more metabolite levels are quantified to result in a metabolite profile and in step b) the metabolite profile is compared to a reference metabolite profile, wherein the reference metabolite profile results from two or more reference values and in step c) the difference between the profile of metabolites and to a reference metabolite profile indicates a higher risk of prevalence of a thoracic aortic aneurysms in the subject.

It is believed that a metabolite profile might outperform the methods relying on a single metabolite level as marker. E.g. the profiles might allow to have similar performance with a reduced number of individually quantified metabolite levels. A metabolite profile allows to include more information and especially for a method of typing different TAA types, such as BAV-A, TAV-A and DISS. The pattern formed by different metabolites results in an additional value. Wherein two diagnoses type might differ quantitatively in a fist metabolite level or the level for a metabolite class, which allows to distinguish them and have a similar quantitative range for a second metabolite level or the level for a metabolite class. However, the second metabolite level or the level for a metabolite class might be necessary to exclude a further third diagnosis type, which would overlap for one of the first two diagnosis types in the first metabolite level.

Thus, in one aspect of the invention provides a method for diagnosing a thoracic aortic aneurysm comprising the steps of a) quantifying two or more metabolite levels are quantified to result in a metabolite profile in a sample from a subject, b) comparing the metabolite profile in the sample with a reference metabolite profile, wherein the reference metabolite profile is obtained by quantifying two or more metabolite levels determined in step a) in a sample from at least one healthy control subject, and c) determining if there is a difference of the metabolite profile to the reference metabolite profile, wherein a difference between the profile of metabolites and to a reference metabolite profile indicates a higher risk of prevalence of a thoracic aortic aneurysms in the subject.

A difference between two profiles can be calculated according to different mathematical methods. Alternatively a difference between two profiles may be defined when metabolite profiles of sample and reference differ in a specific number of the metabolite levels contributing to the metabolite profile. E.g. a difference between the profiles of metabolites of a subject and a reference is present when the profiles differ in one, two, three, or four metabolite levels. Preferably, the difference is present when the profiles differ in at least two metabolite levels.

Similarly, the invention provides a method of typing of a thoracic aortic aneurysm comprising the steps of a) quantifying two or more metabolite levels are quantified to result in a metabolite profile in a sample from a subject b) comparing the metabolite profile in the sample with a classifier system, wherein the classifier system is obtained by quantifying two or more metabolite levels quantified to result in a reference metabolite profile as determined in step a) in a sample from at least one control subject showing a thoracic aortic aneurysm of a single diagnostic type, and c) determining the diagnostic type of the thoracic aortic aneurysm based on the classifier system, wherein the diagnostic type is selected out of bicuspid aortic valve-associated aneurysm (BAV), tricuspid aortic valve-associated aneurysm (TAV), or a dissection of the thoracic aorta (Diss).

The two or more metabolite levels might be determined at the same time, e.g. by relying on an analytical technique which supports the parallel measurement of different metabolite levels. Alternatively, it might be preferred to apply analytical techniques for quantifying the different metabolite levels subsequently. This later option might be preferred if a single metabolite level can already give a defined result depending on the value and further metabolite level might be quantified in a follow-up in case the first metabolite level does not define a unique result. Such a strategy can for example be followed in case, a decision tree is given for determining the diagnosis or the type of TAA. Exemplarily, the decision table shown in Example 5 represents a classifier system according to a method of typing a TAA based on a metabolite profile.

The subject being source of the biological sample is the same subject for which the diagnosis determined according to the invention applies. Whereas a human test person is preferred, the invention is not limited to a human subject and may also be applied to other subjects for which diagnosis of aortic aneurysm might be relevant, i.e. also non-human mammals. Test subject and control subject(s) are of the same species.

A subject might be a person for which there is a reasonable suspicion that the person might have anomalies of the aorta and for situation wherein the respective diagnostic method can support a clinical observation. On the other hand the respective method provides a tool for easy investigation of a large number of subjects, thus, it may be used for routine application and stratification of a larger group. In the latter aspect, the results of the method might not only be a defined diagnosis but determine that a specific subject has a higher risk of development of a clinically manifested aneurysm. It may be decided that a subject with a higher risk of prevalence of a thoracic aortic aneurysms should subsequently and/or regularly be subject of alternative diagnostic methods and/or routine repetitions of the present diagnostic method.

In a further preferred embodiment of the method, the difference between the metabolite level and the reference value or the difference between the profile of metabolites and to a reference metabolite profile indicates that the subject has a thoracic aortic aneurysms.

Moreover, in another preferred embodiment the method according to the invention allows for early diagnosis of thoracic aortic aneurysm. Early diagnosis is referring to a diagnosis at a time point, when no diagnosis of TAA was realised by another method. Even more preferably the method allows early diagnosis at a time point, at which time point or before which time point diagnosis by another method, such as imaging method, may not have been possible. Thus, the method allows diagnosis of TAA while other methods might not be applicable to diagnose TAA.

A sample from a subject as used in the invention should be understood to be any biological sample of, relating to the living organisms representing the subject. Preferred examples of a sample may include, but are not limited to blood, cell culture supernatant, blood serum, plasma, tissue, especially tissue or a liquid sample preparation from tissue e.g. by extraction of the thoracic aorta, especially tissue extractions of an area of the ascending thoracic aorta. Preparation of a tissue sample by suitable measures including use of solvents allows for a liquid tissue extraction sample. Preferably, the biological sample is a liquid sample. More preferably, the biological sample is blood, and most preferable human blood. Liquid means a state of matter with definite volume but no definite shape at 25° C, like water.

In another aspect, the invention provides a kit for diagnosing a TAA according to a method of the invention, wherein the kit comprises instructions to prepare a sample from a subject, quantify at least one metabolite level and guidelines for determining a diagnosis including a reference value.

Furthermore, the invention provides a kit for typing a TAA according to a method of the invention, wherein the kit comprises instructions to prepare a sample from a subject, quantify at least one metabolite level and guidelines for determining a diagnosis including a classifier system.

Additionally the kit might also comprise suitable chemical reagents or practical items for performing the quantitative analysis of the metabolite level such as solvents, detecting reagents, control samples, tubes or test strips.

DETAILED DESCRIPTION OF THE INVENTION

The invention is further illustrated by the attached figures and their description as well as a set of examples.

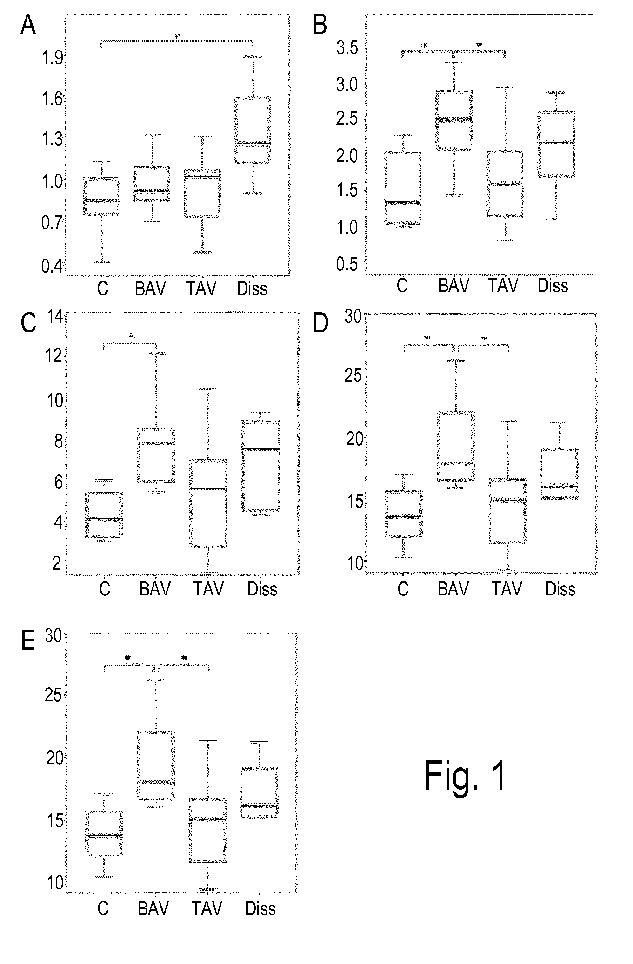

**Figure 1** shows the individual metabolite concentration in $\mu$M of phosphatidylcholine C32:1 **(A),** hydroxylated sphingomyelin C22:2 **(B),** sphingomyelin C18:1 (C), sphingomyelin C22:1 **(D)** and sphingomyelin C24:1 **(E)** for the four different patient groups being the control group (C) or having BAV-associated thoracic aorta aneurysm (BAV), TAV-associated thoracic aorta aneurysm (TAV) or dissection of the thoracic aorta (Diss), wherein * indicates significant with p between 0.01 to 0.05, ** for p in 0.001 to 0.009 and *** for p <0.001.. Box-plots and whiskers are visualized according to Tukey.

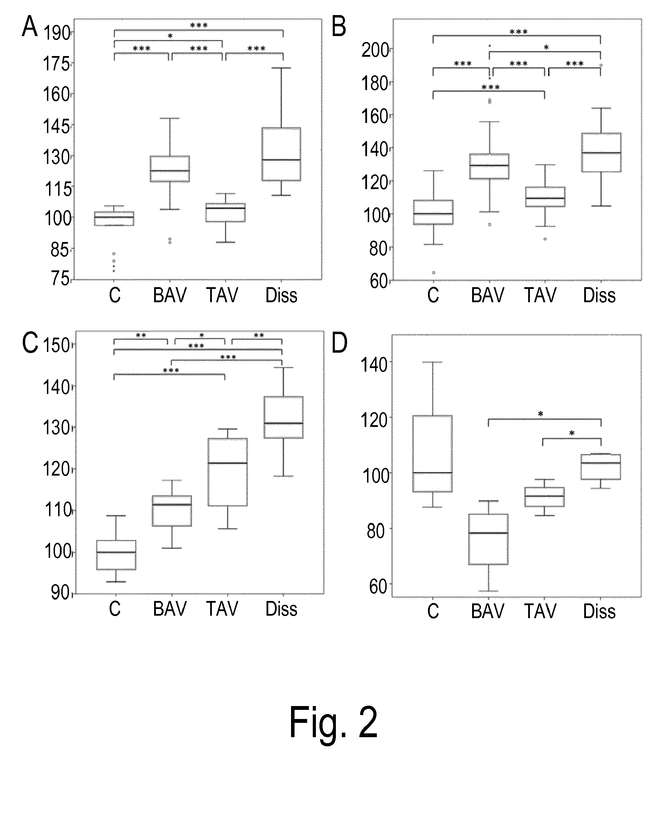

**Figure 2** shows relative values in % for sphingomyelins (n=18) **(A),** glycerophospholipids (n=57) **(B),** amino acids (n=10) **(C),** and ceramides (n=4) **(D)** for the control group (C) in respect to different patient groups having BAV-associated thoracic aorta aneurysm (BAV), TAV-associated thoracic aorta aneurysm (TAV) or dissection of the thoracic aorta (Diss), wherein the individual values results from the median value for each metabolite within a patient group, divided by the overall abundance of this metabolite (sum of medians of four patient groups), and relative to a median ovel all normalized metabolites of the class within the control group (100%). The distributions of metabolites were compared, wherein * indicates significant differences with p between 0.01 to 0.05, ** for p in 0.001 to 0.009 and *** for p <0.001. Box-plots and whiskers are visualized according to Tukey.

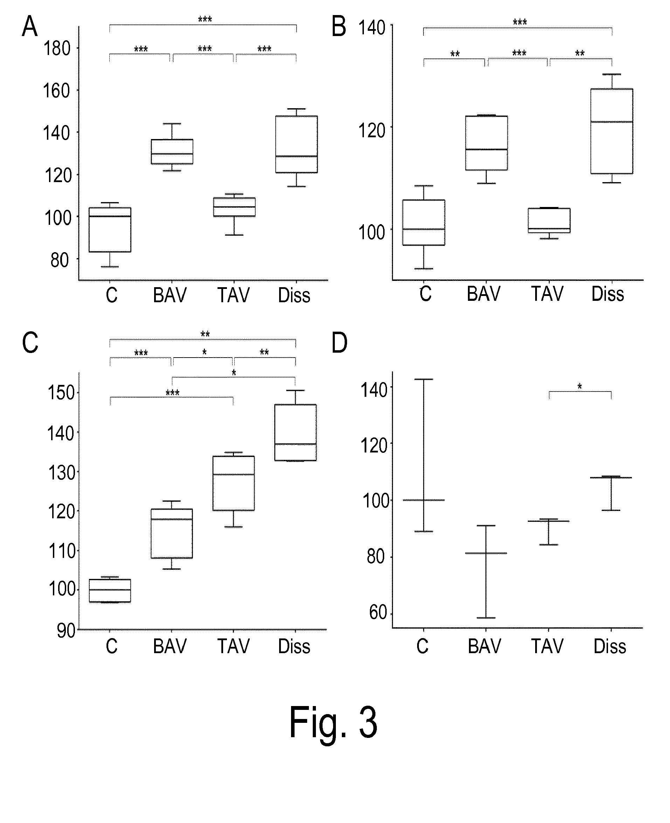

**Figure 3** shows similar to figure 2 pooled metabolite values for sphingomyelins (n=10) **(A),** glycerophospholipids (n=6) **(B),** amino acids (n=5) (C), and ceramides (n=3) **(D),** but with reduced sets of metabolites (n).

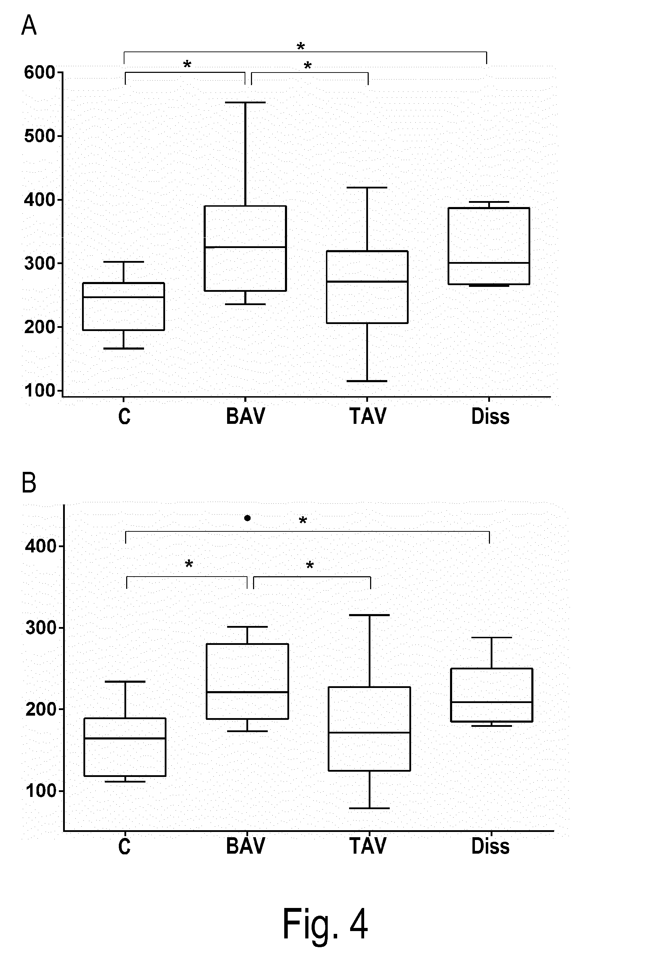

**Figure 4** shows absolute metabolite levels (concentrations in $\mu$M) from the individual samples calculate as sum of 18 sphingomyelin levels **(A)** and 10 selected sphingomyelin levels **(B)** for patients with different diagnostic states, i.e. control (C) with n=8, BAV-associated thoracic aorta aneurysm (BAV) with n=9, TAV-associated thoracic aorta aneurysm (TAV) with n=14 or dissection of the thoracic aorta (Diss) with n=6. Box-plots and whiskers are visualized according to Tukey. * indicates significant differences with p between 0.01 to 0.05.

EXAMPLES

Example 1

**Patient sample**

In this study aortic tissue samples from 37 individuals were analyzed for metabolite profiles. In detail, 8 controls samples stem from organ donors or heart transplant recipients, which had no evidence for aneurysms, atherosclerotic or valvular disease. 9 samples originate from ATAA patients with bicuspid aortic valve (BAV), 14 samples stem from ATAA patients with tricuspid aortic valve (TAV), and 6 samples are collected from patients with an aortic dissection in the ascending thoracic aorta (Diss). All patients showing TAA underwent surgery of the ascending aorta. The patients were selected under the aspect of matching age and sex per group (see Table 1). None of the patients had an inflammatory aortic disease, or suffered systemic connective tissue disorder such as Marfan syndrome or Ehlers Danlos syndrome.

This study has been approved by the Ethics Committee of the Medical University of Innsbruck and the Ethics Committee of the Vienna Medical University confirms and with the Declaration of Helsinki.

Table 1: Overview of clinical patient data involved in the study. The contribution of age, sex and other important clinical facts is given. BSA: body surface area, CAD: coronary artery disease. Age and BSA are given in median $\pm$ standard deviation (SD).

|  | Control n=8 | BAV-A N=9 | TAV-A n=14 | DISS n=6 |
|---|---|---|---|---|
| Male gender | 50.0% | 77.8% | 64.3% | 83.3% |
| Age (years) | 52.9±19.7 | 61.1±9.4 | 62.2±12.5 | 69.0±8.4 |
| BSA (m$^2$) | 1.9±0.2 | 1.9±0.3 | 2.1±0.3 | 1.9±0.2 |
| Hypertension | 37.5% | 77.8% | 58.3%*** | 83.3% |
| Diabetes | 50% | 100% | 23.1%** | 100% |
| CAD | 50% | 25%* | 16.7%*** | 33.3% |
| Hyperlipidemia | 50% | 62.5%* | 46.2%** | 33.3% |
| *data available for 8 patients **data available for 13 patients ***data available for 12 patients | | | | |

All samples analysed originated from the same area of the ascending thoracic aorta and were stored at -80°C immediately after extraction. For metabolite extraction 6 $\mu$L ice cold 100% Ethanol was added per mg tissue and the tubes were placed in a bead-based Mixer Mill MM400 (Retsch, Haan, Germany) for 3 minutes. Afterwards all samples were sonicated (15-20 interval, cycles: 10%, power: MS72/D) and centrifuged twice for 10 min/20,800 g/4°C (Eppendorf, Hamburg, Germany) to remove solid components. Supernatants were collected and stored on dry ice until measurement.

For ceramide and sphingomyelin quantification the samples were prepared according to the following procedure: 20 $\mu$L of sample aliquot, 50 $\mu$L of $H_2O$, 330 $\mu$L of 0.01% BHT in methanol and 670 $\mu$L $CHCl_3$ were placed in a glass vial. The vial was transferred to ultrasonic bath for 60 seconds. Then mixture was vortexed for 30 seconds and incubated for 30 min at room temperature. 300 $\mu$L of water was added to induce the phase separation. After vortexing the mixture for 10 seconds, it was centrifuged for 10 min at 3000 rpm. The lower organic phase was collected and evaporated under the nitrogen. Finally, the lipid extract was dissolved in 200 $\mu$L of hexane/isopropanol/100 mM ammonium acetate (40/50/10, v/v/v).

Example 2

**Quantification of metabolite levels**

20 $\mu$L of supernatant after tissue extraction were prepared for quantification using *AbsoluteID*TM kit p150 (BIOCRATES Life Sciences AG, Innsbruck, Austria). The complete analytical procedure is described in the patent US 2007/0004044 or in the manufacturer's user manual. Briefly, this assay enables the simultaneous quantification of 163 metabolites from four different compound classes: 40 acylcarnitines (Cx:y), hydroxylacylcarnitines (C(OH)x:y), and dicarboxylacylcarnitines (Cx:y-DC), hexose (H1), 14 amino acids, 15 sphingomyelins (SMx:y) and N-hydroxylacyloylsphingosyl-phosphocholine (SM (OH)x:y), 77 phosphatidylcholines (PC, aa=diacyl, ae=acyl-alkyl) and 15 lysophosphatidylcholines. A targeted screening for these metabolites is performed by applying flow injection analysis with tandem mass spectrometric detection (FIA-MS/MS) acquisition with multiple reaction monitoring (MRM) in positive and negative ion mode. Quantification is achieved with internal standards.

The samples were analyzed on a Knauer K-1001 LC pump (Knauer, Berlin, Germany) equipped with a CTC-PAL HTS9 autosampler (CTC Analytics AG, Zwingen, Switzerland) and hyphenated to a QTRAP 3200 mass spectrometer (ABSciex, Toronto, Canada).

Additionally 19 sphingolipids (ceramides and sphingomyelins) were analysed. The quantitative analysis of lipids was performed using flow injection analysis with tandem mass spectrometric detection (FIA - MS/MS). The analysis was carried out on API 4000 QTRAP MS/MS system (AB Sciex Deutschland GmbH, Darmstadt, Germany) coupled to an Agilent 1200 Series HPLC systems and CTC Pal auto sampler (CTC Analytics, Zwingen, Switzerland), all controlled by the Analyst 1.6 software (AB Sciex Deutschland GmbH, Darmstadt, Germany). The FIA - MS/MS analysis was performed using of hexane/isopropanol/100 mM ammonium acetate (40/50/10, v/v/v) as a mobile phase. 10 $\mu$L of the sample extract was injected and the following flow gradient was applied: 0.0 - 1.6 min, 30 $\mu$L/min; 1.6 - 2.4 min, 30 - 200 $\mu$L/min; 2.4 - 2.5 min, 200 - 1500 $\mu$L/min; 2.5 - 4.4 min, 1500 $\mu$L/min; 4.4 - 4.5 min, 30 $\mu$L/min. The MS/MS detection of ceramides and sphingomyelins was performed in negative electro spray ionization mode using multi reaction monitoring (MRM). The ion source parameters were set as follows: curtain gas, 10 arbitrary units; ion source gas 1, 15 arbitrary units, ion source gas 2, 50 arbitrary units; ion source temperature, 250 °C; ion spray voltage, -4500 V; collision gas pressure was set to medium. The dwell time for every MRM was set to 25 msec.

Metabolite concentrations ($\mu$M) were automatically calculated by the MetIDQ software package. Metabolites which showed insufficient measurement stability for a reference sample, which was prepared four times on the plate (relative standard deviation of metabolite concentrations > 30 %) or metabolites which didn't exceed a signal intensity of a minimum of 500 counts, were excluded.

From a total of 182 investigated metabolite levels 92 were found to be detectable and the respective results used for subsequent analysis. They comprise 18, 57, 10, and 4 individually quantified metabolite levels for amino acids, glycerophospholipids, sphingomyelins, and ceramides respectively as well as two carnitines and one hexose.

The individually measured metabolites forming part of the class of amino acids were Glutamine, Glycine, Histidine, Phenylalanine, Proline, Serine, Threonine, Tyrosine, Valine, and Leucine/Isoleucine.

The individually measured metabolites forming the class of glycerophospholipids were Lysophosphatidylcholine acyl C16:0, Lysophosphatidylcholine acyl C17:0, Lysophosphatidylcoline acyl C18:0, Lysophosphatidylcholine acyl C18:1, Lysophosphatidylcholine acyl C18:2, Lysophosphatidylcholine acyl C20:4, Phosphatidylcholine diacyl C18:1, Phosphatidylcholine diacyl C30:0, Phosphatidylcholine diacyl C32:0, Phosphatidylcholine diacyl C32:1, Phosphatidylcholine diacyl C32:2, Phosphatidylcholine diacyl C34:1, Phosphatidylcholine diacyl C34:2, Phosphatidylcholine diacyl C34:3, Phosphatidylcholine diacyl C34:4, Phosphatidylcholine diacyl C36:0, Phosphatidylcholine diacyl C36:1, Phosphatidylcholine diacyl C36:2, Phosphatidylcholine diacyl C36:3, Phosphatidylcholine diacyl C36:4, Phosphatidylcholine diacyl C36:5, Phosphatidylcholine diacyl C36:6, Phosphatidylcholine diacyl C38:3, Phosphatidylcholine diacyl C38:4, Phosphatidylcholine diacyl C38:5, Phosphatidylcholine diacyl C38:6, Phosphatidylcholine diacyl C40:4, Phosphatidylcholine diacyl C40:5, Phosphatidylcholine diacyl C40:6, Phosphatidylcholine diacyl C42:5, Phosphatidylcholine acyl-alkyl C30:1, Phosphatidylcholine acyl-alkyl C32:1, Phosphatidylcholine acyl-alkyl C32:2, Phosphatidylcholine acyl-alkyl C34:0, Phosphatidylcholine acyl-alkyl C34:1, Phosphatidylcholine acyl-alkyl C34:2, Phosphatidylcholine acyl-alkyl C34:3, Phosphatidylcholine acyl-alkyl C36:1, Phosphatidylcholine acyl-alkyl C36:2, Phosphatidylcholine acyl-alkyl C36:3, Phosphatidylcholine acyl-alkyl C36:4, Phosphatidylcholine acyl-alkyl C36:5, Phosphatidylcholine acyl-alkyl C38:2, Phosphatidylcholine acyl-alkyl C38:3, Phosphatidylcholine acyl-alkyl C38:4, Phosphatidylcholine acyl-alkyl C38:5, Phosphatidylcholine acyl-alkyl C38:6, Phosphatidylcholine acyl-alkyl C40:2, Phosphatidylcholine acyl-alkyl C40:3, Phosphatidylcholine acyl-alkyl C40:4, Phosphatidylcholine acyl-alkyl C40:5, Phosphatidylcholine acyl-alkyl C40:6, Phosphatidylcholine acyl-alkyl C42:3, Phosphatidylcholine acyl-alkyl C42:4, Phosphatidylcholine acyl-alkyl C42:4, Phosphatidylcholine acyl-alkyl C44:5, Phosphatidylcholine acyl-alkyl C44:6.

The individually measured metabolites forming the class of sphingomyelins were Sphingomyelin C16:1 with a hydroxy fatty acid, Sphingomyelin C22:1 with a hydroxy fatty acid, Sphingomyelin C22:2 with a hydroxy fatty acid, Sphingomyelin C16:0, Sphingomyelin C16:1, Sphingomyelin C18:0, Sphingomyelin C18:1, Sphingomyelin C20:0, Sphingomyelin C20:2, Sphingomyelin C22:0, Sphingomyelin C22:3, Sphingomyelin C24:0, Sphingomyelin C24:1, Sphingomyelin C26:1, Sphingomyelin C14:0, Sphingomyelin C15:0, Sphingomyelin C17:0, Sphingomyelin C22:1, Sphingomyelin C24:2.

The individually measured metabolites forming the class of ceramides were sphingomyelins forming part of the class sphingomyelins, and Ceramide C14:0, Ceramide C16:0, Ceramide C24:1, Ceramide C24:0.

Regarding the Cx:y nomenclature of the lipids, x indicates the number of carbon atoms in the acyl-moiety or the sum of carbon atoms in acyl- (and alkyl-)moieties attached to the respective head or backbone structure, whereas y indicates the number of double bonds in these moieties.

Example 3

**Determining suitable metabolite levels and reference values by statistical analyses using single compound metabolite levels**

All data resulted from the experiments of Example 2 were analysed using SPSS statistics 22 software (IBM, Armonk, USA). Within groups a possible Gaussian distribution of the data was tested by the Kolmogorov-Smirnov test. Distribution of values of the groups further analyzed was performed by the Student's t-Test. Also the state of variances was considered (Levene's testing). For data with no Gaussian distribution the Mann-Whitney U test was used. Multiple t-Test comparisons were done using ANOVA with Bonferroni post-hoc analysis.

Selected metabolite levels with significant difference to the control group are shown in Figure 1.

These selected metabolites may be suitable for a respective method of typing the diagnostic type for which the difference was observed. A reference value can be determined by the extreme value from the control group or by another value determined from the distribution of values in the control group, e.g., the reference value may be mean+SD or mean-SD.

Example 4

**Pooled metabolite values**

From the metabolite level from example 2, the median of each patient group was normalized to the sum of medians over the four patient groups to achieve a value independent of the absolute concentration. The resulting value was divided by a reference value resulting from the class-wise median of the control group (100%). Therefore, within the control group the individual metabolite values vary around the reference value The analysis yields distributions of the percentage metabolite values representing sphingomyelins (n=18), glycerophospholipids (n=60), amino acids (n=10) and ceramides (n=4) four each of the patient groups as shown in Figure 2.

BAV and DISS showed higher pooled and relative metabolites compared to controls and TAV, regarding sphingomyelins (p<0.001) as well as glycerophospholipids (p<0.001). Although a differentiation between controls and TAV, and BAV and Diss could also be achieved by sphingomyelin and glycerophospholipids comparison (p=0.024 and p<0.001), a more stable differentiation could be achieved by the comparison of amino acid and ceramide contents. TAV values differed significantly from controls by the increased amino acid values (p<0.001) and Diss from BAV by increased ceramide values (p=0.011).

Alternatively, as shown in Figure 3 a reduced set of metabolite values can be used resulting in similar differences for the respective diagnostic groups. For the reduced sets those metabolites were selected from Figure 2, which best reflected the patterns distinguishing the diseases.

The class-wise metabolite values consist of Phenylalanine, Proline, Serine, Threonine, Valine, Leucine, or Isoleucine forming part of the class of amino acids, and Phosphatidylcholine diacyl C34:1, Phosphatidylcholine diacyl C34:2, Phosphatidylcholine diacyl C36:2, Phosphatidylcholine diacyl C36:3, Phosphatidylcholine diacyl C38:3, Phosphatidylcholine acyl-alkyl C36:5 forming part of the class glycerophospholipids, and Sphingomyelin C16:1 with a hydroxyl fatty acid, Sphingomyelin C22:1 with a hydroxyl fatty acid, Sphingomyelin C14:0, Sphingomyelin C15:0, Sphingomyelin C16:0, Sphingomyelin C16:1, Sphingomyelin C18:1, Sphingomyelin C22:0, Sphingomyelin C22:1, Sphingomyelin C24:1, forming part of the class sphingomyelins, and Ceramide C14:0, Ceramide C24:1, Ceramide C24:0 forming part of the class ceramides.

These results show how preferred metabolites reflect a similar distribution of metabolites.

Therefore, a method according to the invention may be further optimized e.g. by limiting the number of individually tested metabolite levels and, thus, alleviating analytical efforts. These reduced lists of metabolites are disclosing preferred metabolites for the methods according to the invention.

Example 5

**Sphingomyelin levels as biomarker to discriminate specific TAA diseases**

The interesting differentiation of the pooled sphingomyelin values observed for BAV and Diss versus control (Figure 3A) as well as significant differences for individual sphingomyelin levels (Figure 2 B-E) motivated for further analysis of this metabolite class.

Sphingomyelin concentrations for 18 measurable sphingomyelin levels from example 2 were summed up for each patient (Figure 4 A). Alternatively, 10 preferred sphingomyelin levels are summed for each patient in Figure 4B, wherein the individual concentrations represent Sphingomyelin C16:1 with a hydroxyl fatty acid, Sphingomyelin C22:1 with a hydroxyl fatty acid, Sphingomyelin C14:0, Sphingomyelin C15:0, Sphingomyelin C16:0, Sphingomyelin C16:1, Sphingomyelin C18:1, Sphingomyelin C22:0, Sphingomyelin C22:1, and Sphingomyelin C24:1. Significant differences were observed for BAV patients versus the control group as well as Diss patients versus the control group as seen in Figure 4 A.

Based on this findings exemplarily a reference value of 210 μM was evaluated as classifier, wherein a summed sphingomyelin level ov above 210 μM indicates an increased risk of BAV.

Table 2: Confusion matrix for a method of identifying an increased risk of BAV based on a sphingomyelin metabolite level

| Prediction | Control | BAV |
|---|---|---|
| Healthy (< 210μM) | 7 | 3 |
| Increased risk of BAV (> 210μM) | 1 | 6 |

Accordingly, the exemplary method of the invention allows to predict an increased risk of a thoracic aortic aneurysm associated to a bicuspid aortic valve. It clearly outperforms a random prediction with the following performance characteristics: precision 85.7%, sensitivity 66.7 %, specificity 70 %, accuracy: 76.5 %.

For calculation of the characteristics, TN is the number of true negatives, TP is the number of true positives, FN is the number of false negatives and TP is the number of false positives and the following formulas apply:

Precision (positive predictive value)

$$TP/(TP+FP) \quad (I)$$

Sensitivity (true positive rate)

$$P/(TP+FN) \quad (II)$$

Specificity (true negative rate)

$$TN/(TN+FP) \quad (III)$$

Accuracy

$$(TP + TN) / (TP+FP+FN+TN) \quad (IV)$$

Example 6

**Classifier systems using a class-wise metabolite level profile**

Alternative classifier systems could rely on the metabolite profile as obtained for the four class-wise metabolite levels (Figure 2). A respective decision table is given in Table 4.

Table 3: Decision table for method of diagnosing and typing TAA based on a metabolite profile comprising the four class-wise metabolite levels

| | Observed metabolite profile pattern | | | |
|---|---|---|---|---|
| Prediction | Sphingomyelins | Glycerophospholipids | Amino acids | Ceramides |
| No TAA | Low | Low | Low | High |

(continued)

| | Observed metabolite profile pattern | | | |
|---|---|---|---|---|
| Prediction | Sphingomyelins | Glycerophospholipids | Amino acids | Ceramides |
| BAV-A | High | High | Middle | Low |
| TAV-A | Low | Low | High | Middle |
| DISS | High | High | Very high | High |

**Claims**

1. Method for diagnosing a thoracic aortic aneurysm comprising the steps of

   a) quantifying a metabolite level based on at least one metabolite in a sample from a subject,
   b) comparing the metabolite level in the sample with a reference value, wherein the reference value is obtained by quantifying the metabolite level determined in step a) in a sample from at least one healthy control subject, and
   c) determining if there is a difference of the metabolite level to the reference value,
   wherein a difference between the metabolite level and the reference value indicates a higher risk of prevalence of a thoracic aortic aneurysms in the subject.

2. Method of typing of a thoracic aortic aneurysm comprising the steps of

   a) quantifying a metabolite level based on at least one metabolite in a sample from a subject,
   b) comparing the metabolite level in the sample with reference values compiled in a classifier system, wherein the classifier system is based on at least one reference value, wherein each reference value is obtained by quantifying the metabolite level determined in step a) in a sample from at least one control subject showing the thoracic aortic aneurysm of a single diagnostic type, and
   c) determining the diagnostic type of the thoracic aortic aneurysm based on the classifier system,
   wherein the diagnostic type is selected out of bicuspid aortic valve-associated aneurysm (DAV-A), tricuspid aortic valve-associated aneurysm (TAV-A), or a dissection of the thoracic aorta (DISS).

3. Method according to claim 1 or 2, wherein the at least one metabolite is selected out of the group comprising representatives out of the classes amino acids, glycerophospholipids, sphingomyelins or ceramides.

4. Method according to claim 1 or 2, wherein the metabolite level of at least one metabolite results from summing up the individual levels of two or more metabolites.

5. Method according to claim 4, wherein the individual levels of two or more metabolites are selected out of a single specific class of metabolites, wherein the specific class is selected out the group comprising amino acids, glycerophospholipids, sphingomyelins or ceramides.

6. Method for diagnosing a thoracic aortic aneurysm comprising the steps of

   a) quantifying two or more metabolite levels are quantified to result in a metabolite profile in a sample from a subject,
   b) comparing the metabolite profile in the sample with a reference metabolite profile, wherein the reference metabolite profile is obtained by quantifying two or more metabolite levels determined in step a) in a sample from at least one healthy control subject, and
   c) determining if there is a difference of the metabolite profile to the reference metabolite profile,
   wherein a difference between the profile of metabolites and to a reference metabolite profile indicates a higher risk of prevalence of a thoracic aortic aneurysms in the subject.

7. Method of typing of an thoracic aortic aneurysm comprising the steps of

   a) quantifying two or more metabolite levels are quantified to result in a metabolite profile in a sample from a subject

b) comparing the metabolite profile in the sample with a classifier system, wherein the classifier system is obtained by quantifying two or more metabolite levels quantified to result in a reference metabolite profile as determined in step a) in a sample from at least one control subject showing a thoracic aortic aneurysm of a single diagnostic type, and

c) determining the diagnostic type of the thoracic aortic aneurysm based on the classifier system,

wherein the diagnostic type is selected out of bicuspid aortic valve-associated aneurysm (DAV-A), tricuspid aortic valve-associated aneurysm (TAV-A), or a dissection of the thoracic aorta (DISS).

8. Method according to claim 6 or 7, wherein at least one metabolite level contributing to the metabolite profile results from summing up individual levels of two or more metabolites.

9. Method according to claim 6 or 7, wherein at least one metabolite level contributing to the metabolite profile results from summing up individual levels of more than one metabolites selected from a single specific class of metabolites for each metabolite level, wherein the specific class is selected out of the group comprising amino acids, glycerophospholipids, sphingomyelins, or ceramides.

10. Method according to claim 6 or 7, wherein the metabolite profile comprises metabolite levels for at least two of the four classes amino acids, glycerophospholipids, sphingomyelins and ceramides resulting from summing up individual levels of more than one metabolite selected from each class.

11. Method according to any one of claim 1 to 10, wherein the at least one wherein the metabolites are selected from the group comprising Glutamine, Glycine, Histidine, Phenylalanine, Proline, Serine, Threonine, Tyrosine, Valine, Leucine, or Isoleucine

forming part of the class of amino acids, and

Lysophosphatidylcholine acyl C16:0, Lysophosphatidylcholine acyl C17:0, Lysophosphatidylcoline acyl C18:0, Lysophosphatidylcholine acyl C18:1, Lysophosphatidylcholine acyl C18:2, Lysophosphatidylcholine acyl C20:4, Phosphatidylcholine diacyl C18:1, Phosphatidylcholine diacyl C30:0, Phosphatidylcholine diacyl C32:0, Phosphatidylcholine diacyl C32:1, Phosphatidylcholine diacyl C32:2, Phosphatidylcholine diacyl C34:1, Phosphatidylcholine diacyl C34:2, Phosphatidylcholine diacyl C34:3, Phosphatidylcholine diacyl C34:4, Phosphatidylcholine diacyl C36:0, Phosphatidylcholine diacyl C36:1, Phosphatidylcholine diacyl C36:2, Phosphatidylcholine diacyl C36:3, Phosphatidylcholine diacyl C36:4, Phosphatidylcholine diacyl C36:5, Phosphatidylcholine diacyl C36:6, Phosphatidylcholine diacyl C38:3, Phosphatidylcholine diacyl C38:4, Phosphatidylcholine diacyl C38:5, Phosphatidylcholine diacyl C38:6, Phosphatidylcholine diacyl C40:4, Phosphatidylcholine diacyl C40:5, Phosphatidylcholine diacyl C40:6, Phosphatidylcholine diacyl C42:5, Phosphatidylcholine acyl-alkyl C30:1, Phosphatidylcholine acyl-alkyl C32:1, Phosphatidylcholine acyl-alkyl C32:2, Phosphatidylcholine acyl-alkyl C34:0, Phosphatidylcholine acyl-alkyl C34:1, Phosphatidylcholine acyl-alkyl C34:2, Phosphatidylcholine acyl-alkyl C34:3, Phosphatidylcholine acyl-alkyl C36:1, Phosphatidylcholine acyl-alkyl C36:2, Phosphatidylcholine acyl-alkyl C36:3, Phosphatidylcholine acyl-alkyl C36:4, Phosphatidylcholine acyl-alkyl C36:5,

Phosphatidylcholine acyl-alkyl C38:2, Phosphatidylcholine acyl-alkyl C38:3, Phosphatidylcholine acyl-alkyl C38:4, Phosphatidylcholine acyl-alkyl C38:5, Phosphatidylcholine acyl-alkyl C38:6, Phosphatidylcholine acyl-alkyl C40:2, Phosphatidylcholine acyl-alkyl C40:3, Phosphatidylcholine acyl-alkyl C40:4, Phosphatidylcholine acyl-alkyl C40:5, Phosphatidylcholine acyl-alkyl C40:6, Phosphatidylcholine acyl-alkyl C42:3, Phosphatidylcholine acyl-alkyl C42:4, Phosphatidylcholine acyl-alkyl C42:4, Phosphatidylcholine acyl-alkyl C44:5, Phosphatidylcholine acyl-alkyl C44:6

forming part of the class glycerophospholipids, and

Sphingomyelin C16:1 with a hydroxy fatty acid, Sphingomyelin C22:1 with a hydroxy fatty acid, Sphingomyelin C22:2 with a hydroxy fatty acid, Sphingomyelin C16:0, Sphingomyelin C16:1, Sphingomyelin C18:0, Sphingomyelin C18:1, Sphingomyelin C20:0, Sphingomyelin C20:2, Sphingomyelin C22:0, Sphingomyelin C22:3, Sphingomyelin C24:0, Sphingomyelin C24:1, Sphingomyelin C26:1, Sphingomyelin C14:0, Sphingomyelin C15:0, Sphingomyelin C17:0, Sphingomyelin C22:1, Sphingomyelin C24:2,

forming part of the class sphingomyelins, and

Ceramide C14:0, Ceramide C16:0, Ceramide C24:1, Ceramide C24:0 forming part of the class ceramides.

12. Method according to claim 11, wherein the metabolites are selected from the group comprising

Phenylalanine, Proline, Serine, Threonine, Valine, Leucine, or Isoleucine forming part of the class of amino acids and

Phosphatidylcholine diacyl C34:1, Phosphatidylcholine diacyl C34:2, Phosphatidylcholine diacyl C36:2, Phosphatidylcholine diacyl C36:3, Phosphatidylcholine diacyl C38:3, Phosphatidylcholine acyl-alkyl C36:5. forming part of the class glycerophospholipids, and

Sphingomyelin C16:1 with a hydroxyl fatty acid, Sphingomyelin C22:1 with a hydroxyl fatty acid, Sphingomyelin C14:0, Sphingomyelin C15:0, Sphingomyelin C16:0, Sphingomyelin C16:1, Sphingomyelin C18:1, Sphingomyelin C22:0, Sphingomyelin C22:1, Sphingomyelin C24:1forming part of the class sphingomyelins, and
Ceramide C14:0, Ceramide C24:1, Ceramide C24:0
forming part of the class ceramides.

13. Method according to any one of claims 1 to 12, wherein the difference between the metabolite level and the reference value or the difference between the profile of metabolites and to a reference metabolite profile indicates that the subject has a thoracic aortic aneurysms.

14. Method according to any one of claim 1 to 13, wherein the method allow for early diagnosis of thoracic aortic aneurysm.

15. Method according to any one of claims 1 to 14, wherein thoracic aortic aneurysms is associated to the ascending aorta.

Fig. 1

Fig. 2

Fig. 3

Fig. 4

**EP 3 171 173 A1**

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | IKONOMIDIS JOHN S ET AL: "Plasma biomarkers for distinguishing etiologic subtypes of thoracic aortic aneurysm disease", JOURNAL OF THORACIC AND CARDIOVASCULAR SURGERY, MOSBY-YEAR BOOK, INC., ST. LOUIS, MO, US, vol. 145, no. 5, 11 January 2013 (2013-01-11), pages 1326-1333, XP028580017, ISSN: 0022-5223, DOI: 10.1016/J.JTCVS.2012.12.027 | 1-15 | INV. G01N33/68 G01N33/92 |
| Y | * the whole document * <br> * abstract * <br> * page 1330, column 1 * <br> * page 1332, column 2, paragraph 2 * | 1-15 | |
| X | GUIDO H.W. VAN BOGERIJEN ET AL: "Biomarkers in TAA-The Holy Grail", PROGRESS IN CARDIOVASCULAR DISEASES., vol. 56, no. 1, 1 July 2013 (2013-07-01), pages 109-115, XP055261200, US ISSN: 0033-0620, DOI: 10.1016/j.pcad.2013.05.004 | 1-15 | |
| Y | * the whole document * <br> * page 110, column 2, paragraph 4 * <br> * page 111, column 2, paragraph 2 * <br> * table 1 * | 1-15 | |

TECHNICAL FIELDS SEARCHED (IPC)

G01N

-/--

The present search report has been drawn up for all claims

| Place of search | Date of completion of the search | Examiner |
|---|---|---|
| The Hague | 4 April 2016 | Jenkins, Gareth |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

# EUROPEAN SEARCH REPORT

Application Number

EP 15 19 5509

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ZUZANA GULASOVÁ ET AL: "Monitoring of Thoracic Aortic Aneurysm in Blood by Fluorescence Spectroscopy", AMERICAN JOURNAL OF MEDICAL AND BIOLOGICAL RESEARCH, vol. 3, no. 5, 2 August 2015 (2015-08-02), pages 128-132, XP055261201, ISSN: 2328-4080, DOI: 10.12691/ajmbr-3-5-2 | 1-15 | |
| Y | * the whole document * * figures 2, 3 * | 1-15 | |
| Y,D | MICHAL CIBOROWSKI ET AL: "Metabolites Secreted by Human Atherothrombotic Aneurysms Revealed through a Metabolomic Approach", JOURNAL OF PROTEOME RESEARCH., vol. 10, no. 3, 4 March 2011 (2011-03-04), pages 1374-1382, XP055261202, US ISSN: 1535-3893, DOI: 10.1021/pr101138m * the whole document * * abstract * * tables 1, 2 * | 1-15 | TECHNICAL FIELDS SEARCHED (IPC) |

The present search report has been drawn up for all claims

| Place of search | Date of completion of the search | Examiner |
|---|---|---|
| The Hague | 4 April 2016 | Jenkins, Gareth |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 2

Europäisches
Patentamt

European
Patent Office

Office européen
des brevets

---

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

---

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☒ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION
SHEET B**

Application Number

EP 15 19 5509

The Search Division considers that the present European patent application does not comply with the requirements of unity of invention and relates to several inventions or groups of inventions, namely:

1. claims: 1-15

   Diagnosing TAA using a metabolic biomarker.

1.1. claims: 1-15(partially)

   Diagnosing TAA using glutamine as a metabolic biomarker.

1.2. claims: 1-15(partially)

   Diagnosing TAA using any of the other metabolic biomarkers listed in claim 11, wherein each biomarker corresponds to a separate invention (i.e. approximately 75 sub-inventions total).

   ---

Please note that all inventions mentioned under item 1, although not necessarily linked by a common inventive concept, could be searched without effort justifying an additional fee.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2010127381 A
- WO 2009091581 A
- WO 2007103568 A
- WO 2014004889 A2
- US 20070004044 A

### Non-patent literature cited in the description

- **PETERS DG ; KASSAM AB ; YONAS H ; O'HARE EH ; FERRELL RE ; BRUFSKY AM.** Comprehensive transcript analysis in small quantities of mRNA by SAGE-lite. *Nucleic acids research,* 1999, vol. 27, e39
- **TUNG WS ; LEE JK ; THOMPSON RW.** Simultaneous analysis of 1176 gene products in normal human aorta and abdominal aortic aneurysms using a membrane-based complementary DNA expression array. *Journal of vascular surgery,* 2001, vol. 34, 143-50
- **CIBOROWSKI M ; MARTIN-VENTURA JL ; MEIL-HAC O ; MICHEL JB ; RUPEREZ FJ ; TUNON J ; EGIDO J ; BARBAS C.** Metabolites secreted by human atherothrombotic aneurysms revealed through a metabolomic approach. *Journal of proteome research.,* 2011, vol. 10, 1374-82
- **CIBOROWSKI M ; BARBAS C.** Metabolites secreted by human atherothrombotic aneurysm. *Methods in molecular biology,* 2013, vol. 1000, 103-13
- **CIBOROWSKI M ; TEUL J ; MARTIN-VENTURA JL ; EGIDO J ; BARBAS C.** Metabolomics with LC-QTOF-MS permits the prediction of disease stage in aortic abdominal aneurysm based on plasma metabolic fingerprint. *PloS one,* 2012, vol. 7, e31982
- **MATT P ; VON ORELLI A ; BERNET F ; GRUSSEN-MEYER T ; LEFKOVITS I ; ZERKOWSKI HR.** Proteomics of ascending aortic aneurysm with bicuspid or tricuspid aortic valve. *Asian cardiovascular & thoracic annals,* 2007, vol. 15, 185-90
- **BLACK KM ; MASUZAWA A ; HABGERG RC ; KHABBAZ KR ; TROVATO ME ; RETTAGLIATI M ; BHASIN MK ; DILLON ST ; LIBERMANN TA ; TOURNPOULIS IK.** Preliminary biomarkers for identification of human ascending thoracic aortic aneurysm. *Journal of The Amercian Heart Association,* 2013, 2